(19) Europäisches Patentamt
European Patent Office
Office européen des brevets

(11) Publication number: **0 101 588**
**B1**

(12) # EUROPEAN PATENT SPECIFICATION

(45) Date of publication of patent specification: **22.07.87**

(51) Int. Cl.[4]: **C 07 C 101/04,**
A 61 K 31/195, A 01 N 37/44

(21) Application number: **83107702.9**

(22) Date of filing: **04.08.83**

(54) **Compositions comprising amino and diamino acid derivatives and use thereof as plant growth stimulants.**

(30) Priority: **13.08.82 IT 2284082**

(43) Date of publication of application:
**29.02.84 Bulletin 84/09**

(45) Publication of the grant of the patent:
**22.07.87 Bulletin 87/30**

(84) Designated Contracting States:
**AT BE CH DE FR GB LI LU NL SE**

(56) References cited:
**DE-A-1 570 903**
**US-A-2 562 198**
**US-A-2 607 797**

**JOURNAL OF THE AMERICAN CHEMICAL SOCIETY, vol. 93, no. 21, October 1971, pp. 5542-5551, WashingtonD .C. (US); G.L.ROWLEY et al.: "On the specificity of creatine kinase. New glycocyamines and glycocyamine analogs related to creatine"**

**CHEMICAL ABSTRACTS, vol. 78, no. 9, 5th March 1973, no. 54806w, Columbus, Ohio (US); J.KYTE: "Titration of the cardiac glycoside binding site of the (Na+ K+)-adenosinetriphosphatase"**

(73) Proprietor: **S.I.P.C.A.M. S.p.A. Società Italiana Prodotti Chimici e per l'Agricoltura Milano**
**Viale Gian Galeazzo, 3**
**I-20136 Milano (IT)**

(72) Inventor: **Ciocca, Baldo**
**Via De Amicis, 33**
**Milano (IT)**
Inventor: **Formigoni, Attilio**
**Via Pietro Micca, 11**
**Legnano (Milano) (IT)**

(74) Representative: **Vatti, Paolo, Dr. Ing. et al**
**Fumero - Studio Consulenza Brevetti**
**Widenmayerstrasse 4/I**
**D-8000 München 22 (DE)**

(56) References cited:

**CHEMICAL ABSTRACTS, vol. 94, no. 23, 8th June 1981, pp. 6,7, no. 185281z, Columbus, Ohio (US); H.D.DELL et al.: "Contribution to the determination of 2-oxopyrrolidine derivatives in biological material"**

**CHEMICAL ABSTRACTS, vol. 92, no. 3, 21st January 1980, p. 274, no. 17742r, Columbus, Ohio (US); J.W.ENGELSMA et al.: "The oxidation of lysinoalanine by L-amino acid oxidase. Identification of products"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 101 588 B1

**Description**

Amino acids are considerably important and active compounds in numerous biological processes in both the vegetable and the animal kingdom.

The physical-chemical properties of numerous amino acid derivatives are still quite unknown, equally as their real biological importance.

The object of the present invention is to use, after preparation by means of a very simple and economical process, amino and diamino acid derivatives having high biological activity in some special fields of use.

It has in fact been discovered that compounds having the formula:

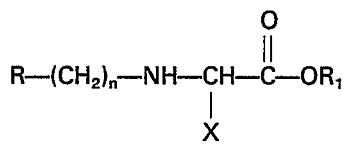 (I)

where:

R can be $-NH_2$ or

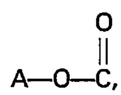

A being hydrogen, an alkali metal, an ammonium group or an aliphatic radical;

n is a whole number between 2 and 5;

X is hydrogen or an alkyl radical with up to 4 carbon atoms;

$R_1$ is hydrogen, an alkali metal, an amino or ammonium group or an aliphatic radical;

can be used successfully as plant growth stimulants.

These compounds can be prepared by known methods as condensation of diamines or of omega-amino acids, or of their lactams or aliphatic esters, with alpha-halogenated aliphatic acids or with their aliphatic esters. In the above described process, N-diamines having the amino radicals at either end of the aliphatic chain and N-omega-amino acids also having the amino radical and the carboxyl radical at the two ends of the aliphatic chain will be used.

Table 1, which follows, lists by way of example only, some compounds which can be used in compositions as plant growth stimulants.

TABLE 1

Examples of amino and diamino acids and their derivatives having the formula (I):

$$R-(CH_2)_n-NH-\underset{\displaystyle X}{\underset{|}{CH_2}}-\overset{\displaystyle O}{\overset{\|}{C}}-OR_1$$

| Compound number | R | n | X | $R_1$ |
|---|---|---|---|---|
| 1 | HOOC— | 3 | —H | —H |
| 2 | NaOOC— | 3 | —H | —Na |
| 3 | KOOC— | 3 | —H | —K |
| 4 | $NH_4$—OOC— | 3 | —H | —$NH_4$ |
| 5 | $(CH_3)_2NH$—HOOC | 3 | —H | —H · $HN(CH_3)_2$ |
| 6 | HOOC | 3 | —$CH_3$ | —H |
| 7 | HOOC | 3 | —$CH_2CH_3$ | —H |
| 8 | HOOC | 2 | —H | —H |
| 9 | HOOC | 2 | —$CH_3$ | —H |
| 10 | HOOC | 4 | —H | —H |
| 11 | HOOC | 4 | —$CH_3$ | —H |
| 12 | HOOC | 5 | —H | —H |
| 13 | HOOC | 5 | —$CH_3$ | —H |
| 14 | $NH_2$— | 3 | —H | —H |
| 15 | $NH_2$— | 4 | —H | —H |
| 16 | $NH_2$— | 4 | —$CH_3$ | —H |
| 17 | $NH_2$ | 4 | —$CH_2CH_3$ | —H |
| 18 | $H_3C$—OOC— | 3 | —H | —$CH_3$ |
| 19 | $H_3CH_2C$—OOC— | 3 | —H | —$CH_2CH_3$ |
| 20 | $H_3C$—OOC— | 3 | —$CH_3$ | —$CH_3$ |
| 21 | $H_3C$—$H_2C$—OOC— | 3 | —$CH_3$ | —$CH_2CH_3$ |
| 22 | H—n$(CH_2)_8$—OOC— | 3 | —H | —n$(CH_2)_8$—H |
| 23 | H—n$(CH_2)_{12}$—OOC— | 3 | —H | —n$(CH_2)_{12}$—H |

Compositions according to the invention, which are preferred, comprise compounds which have the following formulas:

Compound 1) $HOOC-(CH_2)_3-NH-CH_2-COOH$

Compound 6) $HOOC-(CH_2)_3-NH-\underset{\displaystyle CH_3}{\underset{|}{CH}}-COOH$

Compound 12) $HOOC-(CH_2)_5-NH-CH_2-COOH$

Compound 15) $NH_2-(CH_2)_4-NH-CH_2-COOH$

Compound 16) $NH_2-(CH_2)_4-NH-CH(CH_3)-COOH$

Other compositions preferred for the purposes of the invention comprise the ammonium and alkali metal salts, or the aliphatic mono or diesters of the abovementioned compounds. Formula (I) compounds may be identified qualitatively by thin layer chromatography and quantitatively, either by titration with alkali of their acid derivatives or by separation after dansylation.

Formula (I) compounds are particularly suitable for use as plant growth stimulants, both in the early stages of growth of various crops, and also in a more advanced stage on herbaceous or arboreal crops for the purpose of obtaining economically useful results, such as more vigorous and/or more productive plants.

As vegetable crop stimulants, the formula (I) compounds may be used as aqueous solutions or as solutions in solvents emulsifiable in water or even as wettable or dry powders, according to techniques well-known to the expert in the field, for distribution by wet or dry spraying or irrigation on the crops or soil concerned, or for treating seeds before sowing.

Mixture with surfactants or penetrants or various coadjuvants, to facilitate contact with the plant surfaces concerned, might be of particular interest.

Particularly useful is the mixture or simultaneous use with various fertilisers either of natural or synthetic origin, for the simultaneous application of the formula (I) compounds object of the invention and fertilisation of the plants either by root or leaf.

Yet another possibility is the mixture of formula (I) compounds with antiparasitic agents or plant growth regulators of various types, combining in a single distribution operation, plant protection and weedkilling or growth regulation, together with crop growth stimulation.

These compounds are best used by foliar application, in association with fertilisers or pesticides or plant regulators but may also be applied to the roots, with or without such products.

The doses of formula (I) compounds will vary both according to the potency of the compound and according to the desired aim, ranging from 1 g/hectare to as much as 100 kg/ha, the most common intermediate range being between 33 g and 5 kg/ha per treatment.

Example 1

In a liquid mixer prepared during agitation the following aqueous solution:

| | |
|---|---|
| N-carboxymethyl-gamma-aminobutyric acid dipotassium salt | 5 kg |
| Monopotassium phosphate | 10 kg |
| Potassium nitrate | 5 kg |
| Urea | 10 kg |
| Sodium ethylenediaminotetraacetate | 1 kg |
| Sodium dioctyl sulfosuccinate | 1 kg |
| Water | 68 kg |

The product is an activated complex fertiliser containing N.P.K. and growth stimulants that can be used in various ways either for spreading on the earth or for foliar spraying on various crops.

Example 2

In a liquid mixer with agitation prepare the following solution:

| | |
|---|---|
| Ethyl diester of N-carboxymethyl-gamma-aminobutyric acid | 10 kg |
| Gibberellic acid containing 90% Isomer $GA_3$ | 1 kg |
| Tween® 20 (1) | 5 kg |
| Isobutyl alcohol | 74 kg |

The product is a solution suitable for spraying as a growth regulator on various crops.
(1)=Surfactant of Soc. Atlas Chemical Industries (Belgium)

Example 3

In an air-conditioned greenhouse sow Radish crops in a soil fertilised with 15 kg/m$^2$ of a granual fertiliser N.P.K. 15.15.15.

When the crop has emerged from the soil treat twice at an interval of 4 days with aqueous solutions containing compounds according to the invention, leaving one lot as untreated control.

On harvesting, the results are as shown in the following table:

| Compound sprayed | Dose per treatment ($g/m^2$) | Radish crop ($g/m^2$) Whole plants | roots only |
|---|---|---|---|
| 1. N-carboxymethyl gamma-amino-butyric acid disodium salt | 0.01 | 350 | 280 |
| 2. N-methylcarboxymethyl-gamma-aminobutyric acid disodium salt | 0.01 | 321 | 262 |
| 3. N-carboxymethyl-1,4-diamino-butane sodium salt | 0.01 | 335 | 272 |
| 4. Untreated control | — | 198 | 165 |

The above figures show the noteworthy growth stimulant effect on the whole plant and on the roots of the compositions covered by the invention.

Example 4

In a field fertilised with 15 kg/100 $m^2$ of granular 15.15.15 fertiliser sow lettuce and spinach.

When the crops have emerged from the soil give 4 weekly treatments of aqueous solutions of the compounds covered by the invention, leaving one lot as untreated control.

On harvesting, the results are as shown in the following table:

| Compound sprayed | Dose per treatment (g/100 $m^2$) | Harvest (kg/100 $m^2$) Lettuce | Spinach |
|---|---|---|---|
| 1. N-carboxymethyl gamma-amino-butyric acid disodium salt | 2 | 26.5 | 12.6 |
| 2. N-methylcarboxymethyl gamma-aminobutyric acid disodium salt | 2 | 24.3 | 11.5 |
| 3. N-carboxymethyl-1,4-diamino-butane sodium salt | 2 | 23.5 | 11.8 |
| 4. Untreated control | — | 18.0 | 8.3 |

Note how the compositions covered by the invention induce a considerable increase in the harvest of the treated crops.

Example 5

In the field of San Marzano tomatoes fertilised with 10 kg/100 $m^2$ of granular N.P.K. 12.5.20 fertiliser give 2 treatments before flowering and 3 weekly treatments after flowering by spraying aqueous solutions of the products listed hereunder.

On harvesting, the results are as shown hereunder.

| Compound sprayed | Dose per treatment (g/100 $m^2$) | Fruit harvest (kg/100 $m^2$) |
|---|---|---|
| 1. N-carboxymethyl gamma-amino butyric acid disodium salt | 3 | 569 |
| 2. N-methylcarboxymethyl gamma-aminobutyric acid disodium salt | 3 | 513 |
| 3. N-carboxymethyl-1,4-diamino-butane sodium salt | 3 | 574 |
| 4. Untreated control | — | 321 |

The compositions covered by the invention clearly possess an effect that increases the harvest.

Example 6

In a vineyard of Italy grapevines aged 8 years, raised by a modified version of the Guyot system with the plants 1 m apart in the rows and 3 m between the rows, give 2 weekly applications before flowering and 6 weekly applications after flowering, using in the compositions according to the invention compounds as mentioned below mixed with fungicide mixtures containing 0.3% zinc ethylenebis (dithiocarbamate) 80% wettable powder and 0.2% wettable sulfur 80%.

On harvesting, the results are as shown in the table hereunder:

| Compound sprayed | Dose g/100 $m^2$ | Grape harvest kg/100 $m^2$ |
|---|---|---|
| 1. N-carboxymethyl gamma-aminobutyric acid disodium salt | 1 | 351 |
| 2. N-methylcarboxymethyl gamma-amino-butyric acid disodium salt | 1 | 320 |
| 3. N-carboxymethyl-1,4-diamino-butane sodium salt | 1 | 345 |
| 4. Untreated control | — | 234 |

Note that the use of the compositions covered by the invention ensured a significant increase in the grape harvest.

**Claims**

1. Composition for the use as plant growth stimulant, comprising in mixture with inert ingredients amino or diamino acid derivatives having the formula

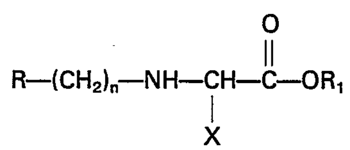 (I)

where:

R can be $NH_2$— or

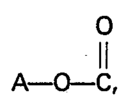

A being hydrogen, and alkali metal an ammonium group or an aliphatic radical;

n is a whole number between 2 and 5;

X is hydrogen or a lower alkyl radical with 1—4 carbon atoms;

$R_1$ is hydrogen, an alkali metal, an amino or ammonium group or an aliphatic radical.

2. Composition as in claim 1) comprising a compound of the formula:

$$HOOC—(CH_2)_3—NH—CH_2—COOH$$

its alkali metal or ammonium salts and aliphatic esters.

3. Composition as in claim 1) comprising a compound of the formula:

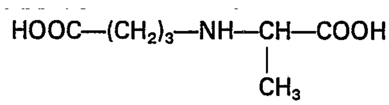

its alkali metal or ammonium salts and aliphatic esters.

4. Composition as in claim 1) comprising a compound of the formula:

$$NH_2—(CH_2)_4—NH—CH_2—COOH$$

its alkali metal or ammonium salts and aliphatic esters.

5. Composition as in claim 1) comprising a compound of the formula:

$$\begin{array}{l} NH_2(CH_2)_4-NH-\underset{\displaystyle\overset{|}{CH_3}}{CH}-COOH \end{array}$$

its alkali metal or ammonium salts and aliphatic esters.

6. Composition as in claim 1) comprising a compound of the formula:

$$HOOC-(CH_2)_5-NH-CH_2-COOH$$

its alkali metal or ammonium salts and aliphatic esters.

7. Process of application of the compounds as in claim 1—6 as plant growth stimulants by distribution of same on the crops, on the soil, or in mixtures on the seed, at doses from 1 g/hectare to 100 kg/ha, preferably from 33 g/ha to 5 kg/ha, per treatment.

8. A compound selected from the group of compounds having the following formulae

$$HOOC-(CH_2)_3-NH-\underset{\displaystyle\overset{|}{CH_3}}{CH}-COOH,$$

$$H_2N-(CH_2)_4-NH-\underset{\displaystyle\overset{|}{CH_3}}{CH}-COOH,$$

$$HOOC-(CH_2)_5-NH-CH_2-COOH,$$

their alkali metal or ammonium salts or their aliphatic esters.

**Patentansprüche**

1. Zusammensetzung zur Verwendung als Pflanzenwachstumsförderer, dadurch gekennzeichnet, daß sie im Gemisch mit Beistoffen Amono- oder Diaminosäurederivate mit der Formel

$$R-(CH_2)_n-NH-\underset{\displaystyle\overset{|}{X}}{CH}-\overset{\displaystyle O}{\overset{||}{C}}-OR_1 \qquad (I)$$

umfaßt, in der:

R $NH_2$ oder

$$A-O-\overset{\displaystyle O}{\overset{||}{C}}$$

sein kann, wobei A Wasserstoff, ein Alkalimetall, eine Ammoniumgruppe oder ein aliphatischer Rest ist;

n eine ganze Zahl zwischen 2 und 5 ist;

X Wasserstoff oder ein niederer Alkylrest mit 1 bis 4 Kohlenstoffatomen ist;

$R_1$ Wasserstoff, ein Alkalimetall, eine Amino- oder Ammoniumgruppe oder ein aliphatischer Rest ist.

2. Zusammensetzung nach Anspruch 1, dadurch gekennzeichnet, daß sie eine Verbindung der Formel:

$$HOOC-(CH_2)_3-NH-CH_2-COOH,$$

deren Alkalimetall- oder Ammoniumsalze und deren aliphatische Ester umfaßt.

3. Zusammensetzung nach Anspruch 1, dadurch gekennzeichnet, daß sie eine Verbindung der Formel:

$$HOOC-(CH_2)_3-NH-\underset{\displaystyle\overset{|}{CH_3}}{CH}-COOH,$$

deren Alkalimetall- oder Ammoniumsalze und deren aliphatische Ester umfaßt.

4. Zusammensetzung nach Anspruch 1, dadurch gekennzeichnet, daß sie eine Verbindung der Formel:

$$NH_2-(CH_2)_4-NH-CH_2-COOH,$$

deren Alkalimetall- oder Ammoniumsalze und deren aliphatische Ester umfaßt.

5. Zusammensetzung nach Anspruch 1, dadurch gekennzeichnet, daß sie eine Verbindung der Formel:

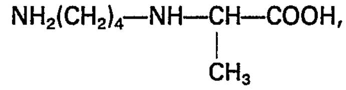

deren Alkalimetall- oder Ammoniumsalze und deren aliphatische Ester umfaßt.

6. Zusammensetzung nach Anspruch 1, dadurch gekennzeichnet, daß sie eine Verbindung der Formel:

$$HOOC—(CH_2)_5—NH—CH_2—COOH,$$

deren Alkalimetall- oder Ammoniumsalze und deren aliphatische Ester umfaßt.

7. Verfahren zur Anwendung der Verbindungen nach Anspruch 1 bis 6 als Pflanzenwachstumsförderer, gekennzeichnet durch Verteilung derselben auf den Feldfrüchten, auf dem Ackerland oder in Gemischen auf der Saat in Dosen von 1 g/ha bis 100 kg/ha, vorzugsweise von 33 g/ha bis 5 kg/ha, pro Behandlung.

8. Verbindung, ausgewählt aus der Gruppe der Verbindungen mit den folgenden Formeln

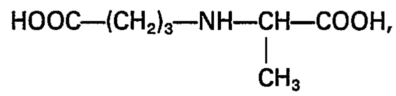

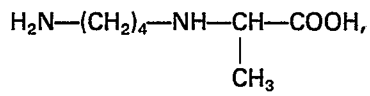

$$HOOC—(CH_2)_5—NH—CH_2—COOH,$$

deren Alkalimetall- oder Ammoniumsalze oder deren aliphatische Ester.

**Revendications**

1. Composition destinée à être utilisée comme stimulant de la croissance des plantes comprenant, en mélange avec des ingrédients inertes, des dérivés d'amino ou de diaminoacides de formule:

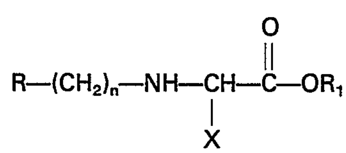 (I)

dans laquelle

R peut être $NH_2$ ou

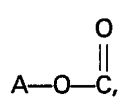

A étant un atome d'hydrogène, un métal alcalin, un groupe ammonium ou un groupe aliphatique;

n est un nombre entier de 2 à 5;

X est un atome d'hydrogène ou un groupe alkyle inférieure de 1 à 4 atomes de carbone;

et $R^1$ est un atome d'hydrogène, un métal alcalin, un groupe amino ou ammonium, ou un groupe aliphatique.

2. Composition suivant la revendication 1, comprenant un composé de formule:

$$HOOC—(CH_2)_3—NH—CH_2—COOH$$

ses sels d'ammonium un d'un métal alcalin, et ses esters aliphatiques.

3. Composition selon la revendication 1, comprenant un composé de formule:

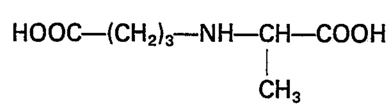

ses sels d'ammonium ou d'un métal alcalin, et ses esters aliphatiques.

4. Composition suivant la revendication 1, comprenant un composé de formule:

$$NH_2-(CH_2)_4-NH-CH_2-COOH$$

ses sels d'ammonium ou d'un métal alcalin, et ses esters aliphatiques.

5. Composition suivant la revendication 1, comprenant un composé de formule:

$$NH_2(CH_2)_4-NH-\underset{\substack{|\\ CH_3}}{CH}-COOH$$

ses sels d'ammonium et d'un métal alcalin, et ses esters aliphatiques.

6. Composition suivant la revendication 1, comprenant un composé de formule:

$$HOOC-(CH_2)_5-NH-CH_2-COOH$$

ses sels d'ammonium ou d'un métal alcalin, et ses esters aliphatiques.

7. Procédé d'application des composés suivant les revendications 1 à 6, en tant que stimulants de croissance des plantes, dans lequel on répartit ceux-ci sur les cultures, sur le sol ou sous la forme de mélanges sur les graines à des doses de 1 g/ha à 100 kg/ha, de préférence de 33 g/ha à 5 kg/ha par traitement.

8. Composé choisi parmi le groupe de composés ayant les formules suivantes:

$$HOOC-(CH_2)_3-NH-\underset{\substack{|\\ CH_3}}{CH}-COOH,$$

$$H_2N-(CH_2)_4-NH-\underset{\substack{|\\ CH_3}}{CH}-COOH,$$

$$HOOC-(CH_2)_5-NH-CH_2-COOH,$$

leurs sels d'ammonium ou d'un métal alcalin, ou leurs esters aliphatiques.